# DEMANDE DE BREVET EUROPEEN

(11) **EP 0 533 569 A1**
(43) Date de publication de la demande: **24.03.1993**
(21) Numéro de dépôt: 92402548.9
(22) Date de dépôt: 17.09.1992
(51) Int. Cl.: C12N 11/04, C02F 1/28

(54) **Procédé de fabrication d'un matériau pour l'épuration des eaux, comprenant des cellules naturelles dans une matrice microporeuse et matériau obtenu**

(30) Priorité: 20.09.1991 FR 9112015
(71) Demandeur: PRONATEC S.A., F-59800 Lille (FR)
(72) Inventeur: Urien, Annie Françoise, F-59790 Ronchin (FR); Christiaen, Daniel, F-59650 Villeneuve d'Ascq (FR); Titeca, Pauline, F-59000 Lille (FR)
(74) Mandataire: Descourtieux, Philippe

(57) **Abrégé**

Le procédé de l'invention concerne la fabrication d'un matériau ayant la forme de billes microporeuses pour l'épuration des eaux. Il consiste :
a) à réaliser un mélange primaire à l'état liquide contenant au moins une résine de préférence époxy, de l'alginate de sodium et des cellules d'origine naturelle dont l'enveloppe extérieure comporte des groupements fonctionnels aptes à retenir des corps dissous dans un milieu liquide, de préférence des algues du type Porphorydium cruentum ou Rhodella reticulata,
b) à verser le mélange primaire, goutte à goutte, dans une solution de chlorure de calcium,
c) à réaliser le durcissement de la résine,
d) et à extraire l'alginate de calcium.

## Description

La présente invention concerne un matériau utilisable pour épurer un milieu liquide, c'est-à-dire un matériau qui comporte des groupements fonctionnels susceptibles de retenir par exemple par échange d'ion, adsorption ou complexation des corps dissous dans ledit milieu. Elle concerne plus particulièrement un procédé de fabrication d' un matériau formant matrice microporeuse à base de résine et comprenant des cellules naturelles.

Parmi les matériaux usités dans le domaine de l'épuration des eaux il y a d'un côté des résines chimiques et de l'autre côté des substances vivantes. Les résines chimiques sont des produits de synthèse dont certains composants comportent des groupements fonctionnels aptes à la retention des corps dissous ; il existe une multiplicité de résines dont le squelette et les groupements fonctionnnels sont adaptés au milieu à traiter. Elles ont généralement la forme de billes et sont mises en oeuvre dans des colonnes qui sont traversées par le milieu à traiter.

Les substances vivantes utilisées en épuration des eaux sont des cellules naturelles dont l'enveloppe extérieure comporte des groupements fonctionnels aptes à la rétention des corps dissous. L'avantage des substances vivantes par rapport aux résines réside bien sûr dans leur capacité à proliférer, à se multiplier naturellement ; cependant pour cela il faut que le milieu, dans lequel elles séjournent, remplisse certaines conditions particulières, notamment de lumière. Les substances vivantes présentent d'autres inconvénients : elles nécessitent un entretien important, elles sont sensibles à des produits et concentrations toxiques et ne sont pas régénérables.

On connaît par la publication dans ENVIRONMENTAL SCIENCE AND TECHNOLOGY, vol. 24 n° 2 Septembre 1990 pages 220-221, qu'il est possible d'immobiliser des algues dans un polymère réticulé en vue de réaliser un matériau pour épuration des eaux. Il s'agissait en l'occurrence de mélanger des algues du type S.quadricauda à de l'éthylacrylate et de l'éthylène glycol, qui réagissent selon la technique de polymérisation en émulsion.

Le but que s'est fixé le demandeur est de proposer un procédé de fabrication d'un matériau pour épuration des eaux du type précité mais dont la matrice d'immobilisation des cellules naturelles soit microporeuse et qui se présente sous forme de billes, dont le calibrage soit parfaitement contrôlable et reproductible, ce que ne permet pas le procédé connu.

Ce but est parfaitement atteint par le procédé de l'invention, qui consiste :
a) à réaliser un mélange primaire à l'état liquide contenant au moins une résine, de l'alginate de sodium et, des cellules d'origine naturelle dont l'enveloppe extérieure comporte des groupements fonctionnels aptes à retenir des corps dissous dans un milieu liquide,
b) à verser le mélange primaire, goutte à goutte, dans une solution de chlorure de calcium,
c) à réaliser le durcissement de la résine,
d) et à extraire l'alginate de calcium.

Au contact de la solution de chlorure calcium le mélange primaire se prend en masse sous forme de billes, du fait de la transformation de l'alginate de sodium liquide en alginate de calcium solide. Cette prise en masse immédiate permet de garder la forme de goutte lors du durcissement de la résine. L'extraction de l'alginate de sodium permet de créer la microporosité adéquate.

Dans le cas d'une résine époxy, le mélange primaire contient également un durcisseur, et le durcissement de la résine est obtenu aprés séchage des gouttes.

L'extraction de l'alginate de calcium peut être obtenue en mettant les billes, obtenues après durcissement de la résine, en contact avec une solution tampon phosphate et/ou d'EDTA (éthylène diamine tétraacétique) ce qui a pour effet de déplacer l'équilibre, d'extraire l'alginate de calcium de la résine et donc de créer la microporosité.

Selon un mode particulier de réalisation, les cellules d'origine naturelle étant des algues rouges unicellulaires à enveloppe extérieure polysaccharidique, le procédé comporte les étapes préalables suivantes :
a) centrifugation des algues vivantes,
b) activation, par exemple par protonation à l'aide d'une solution acide,
c) broyage.

Ces étapes sont destinées à obtenir une nécromasse, c'est-à-dire des algues mortes, sous une forme structurelle et chimique appropriée. L'activation prend, lorsque la fixation est liée à l'échange ionique, la forme d'une protonation. La protonation a pour objet de déplacer les cations naturellement fixés aux groupements anioniques de l'enveloppe polysaccharidique et de les remplacer par des ions H⁺ ; les groupements fonctionnels sont alors disponibles pour retenir d'autres cations qui déplaceront l'ion H⁺, selon l'échelle d'affinité. Le broyage a pour objet de rendre accessible les enveloppes extérieures d'un plus grand nombre de cellules, et d'obtenir lors du mélange une meilleure répartition.

Le demandeur a obtenu des résultats particulièrement performants en mettant en oeuvre, dans le procédé de l'invention, un mélange constitué de :
* résine époxy = a %
* durcisseur = b %
* cellules protonées et broyées = c %
* alginate de sodium à 8 % = d %
* eau = e %

a % est de l'ordre de 10%, b % est compris entre 10 et 12 %, c % entre 15 et 20 %, d % entre 20 et 30 %, e % entre 40 et 45, en poids.

Bien sûr, étant immobilisées dans la résine , les cellules d'origine naturelle ne sont plus à même de se reproduire et de proliférer ; elles sont mortes . Cependant le demandeur a vérifié que, même dans cet état, l'enveloppe extérieure des cellules conserve des groupements fonctionnels aptes à retenir les corps dissous, et que , de plus , des groupements sont parfaitement régénérables comme des résines chimiques traditionnelles.

Les cellules immobilisées dans la résine microporeuse proviennent de produits d'origine végétale ou bactérienne.

Il peut s'agir notamment d'algues, de préférence d'algues rouges unicellulaires.

Le demandeur a retenu en particulier les espèces suivantes: Porphyridium cruentum et Rhodella reticulata, qui sont des algues rouges unicellulaires protégées par une enveloppe polysaccharidique. Les polysaccharides en question sont de type sulfaté et se composent principalement de D-xylose, D-glucuronique, D-glucose, D-et L-galactose et éventuellement de rhamnose. Le matériau ainsi constitué convient particulièrement pour la rétention des corps dissous à l'état cationique, notamment d'ion ammonium et d'ions de métaux lourds en particulier de chrome hexavalent.

La résine dans laquelle sont immobilisées les cellules d'origne naturelle est de préférence une résine époxy. On comprend que la microporosité permet de disposer d'une très grande surface de contact entre le milieu liquide et donc les corps dissous et les groupements fonctionnels des cellules qui sont accessibles en surface de la résine.

La matrice retenue comme support d'immobilisation possède des groupements fonctionnels qui complètent les propriétés électrostatiques des cellules naturelles. En effet, la matrice est neutre vis à vis des espèces cationiques simples c'est à dire qu'elle ne modifie pas la capacité de fixation des cellules immobilisées mais elle permet d'augmenter cette capacité lorsque les espèces ioniques sont plus complexes comme le chrome hexavalent.

L'invention sera mieux comprise à la lecture de la description qui va être faite d'un mode de préparation d'un matériau pour épuration des eaux comportant des cellules naturelles immobilisées dans une résine microporeuse.

On est parti d'algues rouges unicellulaires de l'espèce Porphyridium cruentum . Ces algues possèdent une enveloppe extérieure polysaccharidique. Les polysaccharides en question sont de type sulfaté ; ils se comportent principalement de D - xylose, D - glucuronique, D - glucose, D -et L - galactose, éventuellement de rhamnose. Cette enveloppe extérieure comporte donc des groupements chargés négativement, notamment acides uroniques et sulfates.

Ces algues peuvent être cultivées en bioréacteur dans des poches plastiques ou dans des gaines de plusieurs centaines de litres, par exemple 800 litres, avec un temps de doublement de quelques heures, ce qui permet d'obtenir une biomasse contenant des quantités importantes d'algues vivantes dans des temps très courts. Cette biomasse est centrifugée à grande vitesse pendant un temps suffisant, par exemple de l'ordre de 20 mn, pour obtenir un culot de centrifugation qui a une concentration en cellules de 300 à 600 millions par millilitre.

Les algues ainsi centrifugées ne sont plus à même de vivre et de proliférer, n'étant plus dans un milieu approprié. Il s'agit donc d'algues mortes et l'on peut dire que la biomasse est devenue, après centrifugation, une nécromasse.

La nécromasse est mise en contact avec de l'acide nitrique 0,1 N pendant vingt minutes sous faible agitation. Ce traitement conduit à la protonation des groupements fonctionnels chargés négativement, présents sur l'enveloppe extérieure des cellules, c'est-à-dire la substitution par l'ion H⁺ des cations naturellement liés à ces groupements. Bien sûr les conditions opératoires de la protonation sont fonction de l'acide choisi et de sa concentration.

Après protonation la nécromasse est broyée, puis mélangée avec une résine synthétique, son durcisseur et de l'alginate de sodium. On sait que l'alginate de sodium est soluble dans l'eau, alors que l'alginate de calcium est insoluble.Le mélange liquide est versé goutte à goutte dans une solution de chlorure de calcium ; la transformation de l'alginate de sodium en alginate de calcium solide est instantanée et permet à la goutte de conserver sa structure : on obtient des billes dont le diamètre est de l'ordre de 2 mm. Cependant la viscosité du mélange peut être modifiée pour obtenir une granulométrie différente. Ces billes sont égouttées et séchées, par exemple par air chaud pulsé à 70°C pendant une heure. Dans ce dernier cas, elles ont une coloration brun clair et ne contiennent plus que 30 à 40 % d'eau. Pendant l'opération de séchage, la résine a été prise en masse.

Les billes sont ensuite mises en contact avec une solution de tampon phosphate et/ou d'EDTA. Les ions phosphates présents dans cette solution complexent le calcium et entraînent un relargage de l'alginate. Les emplacements occupés dans les billes par l'alginate de calcium sont ainsi libérés, ce qui crée une microporosité ouverte. Les billes microporeuses obtenues possédent toutes les propriétés recherchées pour le matériau destiné à l'épuration des eaux : perméabilité à l'eau et aux ions, immobilisation des algues mortes, accessibilité des groupements fonctionnels présents sur l'enveloppe extérieure desdites algues.

Les essais menés par le demandeur ont permis de définir des conditions préférées pour le mélange servant à la réalisation de telles billes microporeuses :
* résine époxy = a %
* durcisseur = b %
* cellules protonées et broyées = c %
* alginate de sodium à 8 % = d %
* eau = e %

a % est de l'ordre de 10 %, b % est compris entre 10 et 12 %, c % entre 5 et 20 %, d % entre 20 et 30 %, e % entre 40 et 45 %, en poids. De très bons résultats ont été obtenus avec a % = 10 %, b %= 10 %, c % = 10 %, d % = 30% et e % = 40 %.

Les billes microporeuses, contenant la nécromasse immobilisée, ont été mises en oeuvre pour l'épuration des eaux dans une colonne à contre-courant, dont la partie basse est munie d'une grille de retenue des billes, d'une première entrée pour l'effluent à épurer et d'une seconde entrée pour une alimentation en air comprimé par exemple et dont la partie haute est évasée pour assurer le remplisage de la colonne et comporte une sortie en trop plein pour l'évacuation de l'effluent après que celui-ci ait traversé toute la colonne.

Des essais ont été conduits sur des effluents chargés en chrome hexavalent. L'évolution du chrome hexavalent est suivie par absorption atomique en utilisant le four lorsque les concentrations en chrome total sont de l'ordre du microgramme, par absorption atomique en flamme lorsqu'elle sont de l'ordre du milligramme, par dosage colorimétrique avec le diphényl carbazide lorsque les concentrations en chrome sont comprises titrimétrique avec le sulfate d'ammonium ferreux lorsque les concentrations en chrome sont supérieures en milligramme par litre.

Les résultats obtenus sont les suivants. On a retenu près de 30 mg de chrome hexavalent par gramme de nécromasse sèche immobilisée. Il est à noter que ce résultat est tout-à-fait inattendu dans la mesure où des essais menés en laboratoires avec la nécromasse sèche, mais non immobilisée sous forme de billes microporeuses avaient conduits à une rétention de l'ordre de 17 mg de chrome hexavalent par gramme de nécromasse sèche libre. Ce résultat confirme le rôle actif du support d'immobilisation dans la fixation du chrome.

En ce qui concerne les débits de passage de l'effluent dans la colonne, ils doivent être tels qu'ils permettent un certain temps de contact avec le lit de billes ; des efficacités de rétention de 72, 86 et 89 % ont été obtenus avec des débits de 125, 250 et 1000 millilitres par minute.

La présence du chrome hexavalent dans les effluents est très réglementée. La technique d'épuration mettant en oeuvre le matériau de l'invention constitue une étape de finition du traitement de ces effluents, permettant d'éliminer l'excés de chrome. Par exemple, selon la législation française actuelle, la concentration limite en chrome hexavalent dans les eaux usées est de 100 microgrammes par litre. Grâce à cette technique, ceci peut être atteint sans difficulté.

D'autres essais ont été conduits avec des effluents chargés en ions ammonium. La variation de l'ion ammonium dans l'effluent a été suivie au moyen d'une électrode spécifique à ammoniac. On a obtenu une rétention de 12 mg de NH₄⁺ par gramme de nécromasse sèche incluse dans les billes microporeuses. Comparativement cette rétention était de 15 mg lorsque la nécromasse était libre.

La nécromasse est, après usage, régénérée par contact avec une solution acide. Cette régénération est réalisée par passage de ladite solution acide à contre courant dans la colonne. La nécromasse peut subir plusieurs cycles de régénération sans que cela nuise à son efficacité. De plus, comparativement aux résines chimiques traditionnelles, la régénération de la nécromasse immobilisée dans une matrice microporeuse selon l'invention se fait dans des conditions particulièrement performantes, demandant des volumes d'acide et d'eau de rinçage très nettement inférieurs, par exemple pour la régénération des billes contaminées par l'ammomnium, 0,01 volume d'acide nitrique par volume de billes microporeuses contenant la nécromasse comparé à 8 volumes d'acide chlorhydrique par volume pour une résine de type copolymère de styrène-divinyl-benzène, échangeuse de cations fortement acide.

La régénération de la résine biologique contaminée par le chrome s'effectue par un traitement avec l'acide oxalique, puissant complexant du chrome.

L'invention n'est pas limitée au mode de réalisation qui a été décrit ci-dessus à titre d'exemple non exhaustif.

D'autres cellules d'origine naturelle peuvent être mises en oeuvre dans la réalisation du matériau pour épuration des eaux selon l'invention. Parmi les algues rouges unicellulaires, il peut s'agir en particulier de l'espèce Rhodella reticulata, mais aussi Rhodella maculata, Porphyridium reticulata. On peut envisager d'autres types d'algues, par exemple Chlorella sp. qui est une algue verte, le Lithothamium qui est une microalgue rouge calcaire, ou encore des cellules provenant d'autres substances végétales, par exemple des cuticules extraits de graines de lupin, dont les pectines sont essentiellement constituées d'acides uroniques, de rhamnose et de sucres neutres, ou bien encore des molécules d'origine bactérienne comme des exopolysaccharides bactériens.

On comprend que ce sont les propriétés chimiques de l'enveloppe extérieure des cellules qui sont utilisées dans la présente invention, à savoir la capacité de cette enveloppe, par les groupements fonctionnels qu'elle contient, à retenir les corps dissous à l'état ionique dans le milieu liquide à traiter.

D'autres résines peuvent aussi être utilisées, soit en présence d'un durcisseur, soit susceptibles de se polymériser dans des conditions acceptables pour assurer leur durcissement.

## Revendications

1. Procédé pour la fabrication d'un matériau comportant une matrice en résine, pour l'épuration des eaux caractérisé en ce qu'il consiste :
a) à réaliser un mélange primaire à l'état liquide contenant au moins une résine, de l'alginate de sodium et des cellules d'origine naturelle dont l'enveloppe extérieure comporte des groupements fonctionnels aptes à retenir des corps dissous dans un milieu liquide,
b) à verser le mélange primaire, goutte à goutte, dans une solution de chlorure de calcium,
c) à réaliser le durcissement de la résine,
d) et à extraire l'alginate de calcium.

2. Procédé selon la revendication 1 caractérisé en ce que le mélange primaire contient une résine époxy et son durcisseur et en ce que le durcissement de la résine est obtenu par séchage.

3. Procédé selon la revendication 1 caractérisé en ce que l'extraction de l'alginate de calcium est réalisé par mise en contact avec une solution tampon de phosphate et/ou d'éthylène diamine tétraacétique.

4. Procédé selon la revendication 1 caractérisé en ce que les cellules d'origine naturelle étant des algues rouges unicellulaires à enveloppe extérieure polysaccharidique, il comporte les étapes préalables suivantes :
a) centrifugation des algues vivantes,
b) activation, par exemple protonation à l'aide d'une solution acide,
c) broyage.

5. Procédé selon la revendication 1 caractérisé en ce que le mélange primaire est constitué de :
* résine époxy = a %
* durcisseur = b %
* cellules protonées et broyées = c %
* alginate de sodium à 8 % = d %
* eau = e %
a % est de l'ordre de 10 %, b % est compris entre 10 et 12 %, c % entre 5 et 20 %, d % entre 20 et 30 %, e % entre 40 et 45 %, en poids.

6. Matériau pour épuration, susceptible d'être obtenu par le procédé de la revendication 1, comportant des algues mortes du type Porphorydium cruentum ou Rhodella reticulata immobilisées dans une résine microporeuse en forme de bille.

7. Matériau selon la revendication 6 caractérisée en ce que la résine est une résine époxy.
